Europäisches Patentamt

European Patent Office

Office européen des brevets

(19)

(11) Publication number: **0 210 032 B1**

# EUROPEAN PATENT SPECIFICATION

(12)

(45) Date of publication of patent specification: **18.12.91**    (51) Int. Cl.⁵: **A61F 5/441, A61F 5/445**

(21) Application number: **86305402.9**

(22) Date of filing: **14.07.86**

(54) Ostomy appliances.

(30) Priority: **16.07.85 GB 8517870**
          **19.12.85 GB 8531257**

(43) Date of publication of application:
      **28.01.87 Bulletin  87/05**

(45) Publication of the grant of the patent:
      **18.12.91 Bulletin  91/51**

(84) Designated Contracting States:
      **AT BE CH DE FR GB IT LI LU NL SE**

(56) References cited:
      **FR-A- 2 431 285**
      **GB-A- 1 595 906**
      **GB-A- 2 094 153**
      **US-A- 2 555 086**

(73) Proprietor: **E.R. Squibb & Sons, Inc.**
      **Lawrenceville-Princeton Road**
      **Princeton, N.J. 08540-4000(US)**

(72) Inventor: **Steer, Peter Leslie**
      **Woodlands Rise Kingscote**
      **East Grinstead Sussex(GB)**

(74) Representative: **Cook, Anthony John et al**
      **D. YOUNG & CO. 10, Staple Inn**
      **London, WC1V 7RD(GB)**

Rank Xerox (UK) Business Services

## Description

The present invention relates to ostomy appliances. In particular, the invention relates to a bag side coupling element for use on or with an ostomy bag, and to bags including such elements.

Kingsdown Medical Consultants Limited in British Patent (GB-A-No) 1 595 906 disclosed a surgical dressing which embodied a gas exit path having a gas filter interposed therein. The filter may be located in a coupling element which is secured to the bag. A valve in the gas path permitting the patient to control venting of flatus gases may be included. To the best of our knowledge and belief, this arrangement has not been successful, possibly because of difficulties and expense in manufacture.

According to the present invention, there is provided a bag side coupling element for an ostomy bag, comprising a flat, elongated member including a flange surrounding a stomal orifice and having a coupling portion by means of which it can be coupled to a body side coupling element, said flange having an extension connected thereto, characterised in that the extension has a peripheral wall containing an internal peripheral groove therein, there being spaced inwardly of the wall a circular upraised rib which projects from a base wall of the extension, in that the extension has a central hole through which gas from the bag can enter, and in that the groove is arranged to receive a counterpart rib or rim on a substantially cylindrical wall of a filter cover, a disc filter being locatable within the space defined by the extension and its cover, whereby in use the disc filter is caused to bear on the upraised rib so as to prevent the disc filter being by-passed by gases to be deodorised or filtered.

According to an embodiment of the invention, the filter cover has a plurality of holes therein, the holes being of tapering configuration such that their cross-sectional area at the inner surface of the cover is less than that at the outer surface of the cover.

In an advantageous version of the invention, the filter cover is of substantially oval shape and has flanges which extend beyond the confines of the wall.

According to a preferred embodiment of the invention, the flange and its extension are integrally molded from plastic. According to a specially preferred version of the invention, a disc filter is disposed within a housing whose peripheral wall is formed integrally with the extension.

In another embodiment of the invention, the coupling portion is formed as a so-called "snap lock coupling" in which an interengaging rib and channel effect a connection between a bag-side and a body side ostomy coupling element.

The invention will be better understood from the following non-limiting description of examples thereof given with reference to the accompanying drawings, in which:-

Figure 1 is a diagrammatic rear elevation view of an example of an ostomy bag;

Figure 2 is a vertical cross-section taken on the line II-II in Figure 1 showing the construction of the flange and its attachment to the bag;

Figure 3 is a cross-sectional view of a bag side coupling element;

Figure 4 is an elevational view of the bag side coupling element of Figure 3;

Figure 5 is an elevational view of a bag side coupling element in accordance with the invention for use with an ostomy bag;

Figure 6 is a cross-sectional view of the bag side coupling element of Figure 5;

Figures 7 and 8 are enlarged cross-sectional views of parts of the element shown in Figure 6;

Figure 9 is an elevational view of an alternative embodiment of a bag side coupling element for use with an ostomy bag;

FIG. 10 is a sectional view of an alternative embodiment of a bag side coupling element for use with an ostomy bag;

FIGS. 11 and 12 are cross-sectional views on an enlarged scale of parts of the element shown in FIG. 10;

FIG. 13 is a top plan views of one example of a snap-on filter cover according to the invention which is usable with any one of the coupling elements shown in FIGS. 4, 5, or 9;

FIG. 14 is a central cross-sectional view of the snap-on filter cover shown in FIG. 13; and

FIG. 15 is a side view of the snap-on filter cover shown in FIG. 13.

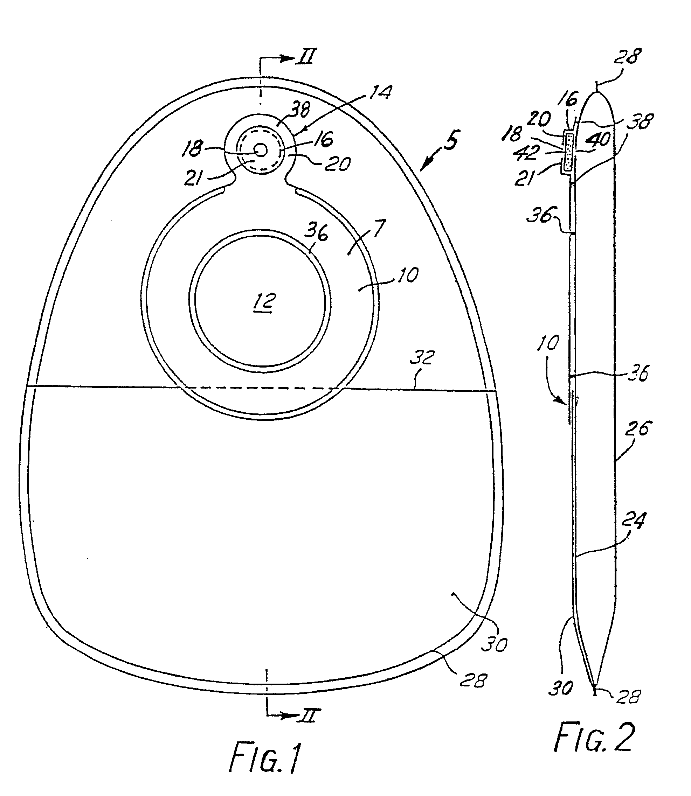

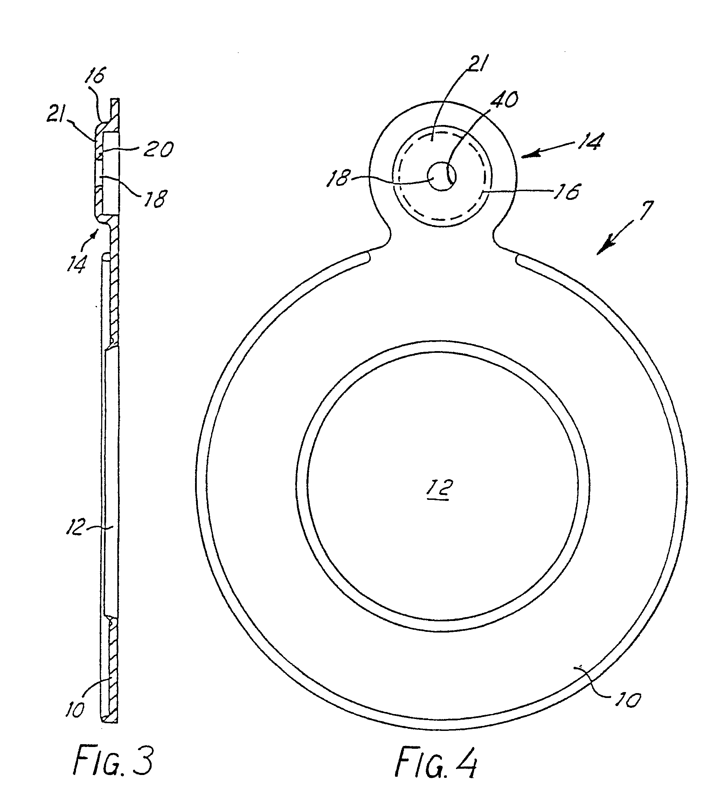

Referring generally to FIGS. 1 and 2, the ostomy bag 5 including the present invention may be better understood by firstly considering the bag-side coupling element 7 illustrated in FIGS. 3 and 4. The bag-side coupling element 7 comprises a flat annular flange 10 having a stomal orifice 12. The flange 10 is integral with an extension 14. The extension 14 is substantially circular, as shown in FIG. 4, and has a shallow cylindrical housing 21 having a peripheral wall 16 and a top wall 20. A hole 18 is formed in a central region of the top wall 20. The purpose of the hole 18 is to allow exit of gases to the atmosphere as explained more fully hereinafter.

The ostomy bag 5 illustrated in FIGS. 1 and 2 has front and rear walls 24, 26, respectively, secured together by a peripheral weld 28. In addition, a perforated sheet 30 extending only over the lower part of the bag 5 is attached to the ostomy bag 5 by the peripheral weld 28. The top edge 32 of the perforated sheet 30 is not attached to the front bag wall 24. One purpose of the perforated sheet 30 is to provide a surface which is less cold to the touch so that when this portion of the ostomy bag 5 contacts the skin of the wearer comfort is improved.

The bag-side coupling element 7 includes a flange 10. The coupling element 7 is secured by welds 36, 38 to the front wall 24 of the ostomy bag 5. Each of these welds 36, 38 is substantially circular. The weld 38 surrounds a hole 40 in an upper region of the front wall 24. The hole 40 is located within the confines of the wall 16 of the shallow cylindrical 21 portion of the bag-side coupling element 5.

The coupling element 7 has a flange 10 which presents a flat surface away from the bag 5, that is to say towards the body-side coupling element with which it is intended to cooperate. A series of adhesive annuli may be used to connect the bag 5 to a body-side ostomy coupling member. The extension 14 of the coupling element 7 is secured to the bag 5 by the weld 38 and it preferably contains a filter pad 42 (see FIG. 2) chosen to be a snug fit within the shallow cylindrical space 21 defined by the walls 16, 20. While reference is being made to separate walls it will be appreciated that the whole bag-side coupling element 7 illustrated in FIG. 3 is made from a single piece of plastic, for example, by injection molding.

In use, the extension 14 stiffens the upper portion of the bag wall 24 and effectively prevents undesired drooping of the bag 5. It also houses the filter 42. The hole 40, the filter 42, and the hole 18 constitute the exit path for any flatus gases which have entered the bag 5 after having been discharged from the stoma of the wearer. Another useful feature of the extension 14 is that it provides a tab which can be gripped by the wearer of the bag 5, thereby facilitating removal of the bag 5 to change it. If it is not desired to provide a filter pad 42, then the hole 40 in the upper region of the bag wall 24 could be omitted.

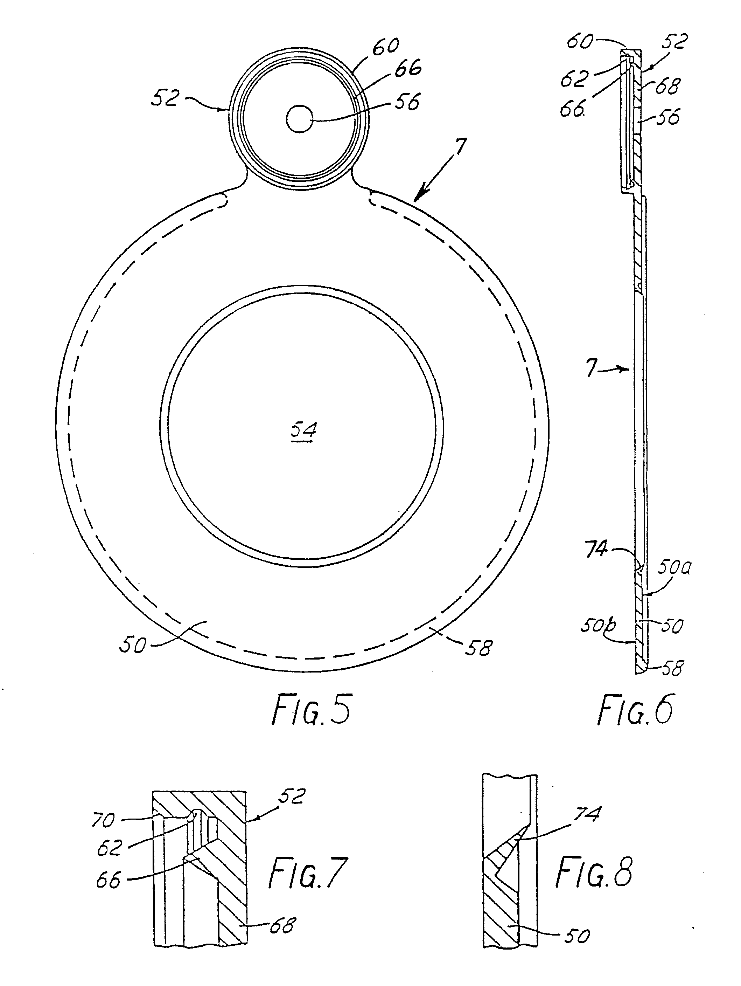

Referring now to FIGS. 5-8, the illustrated bag-side coupling element 7, shown in front view in FIG. 5 and in a sectional view in FIG. 6, has a flange 50 and an extension 52. There is a circular central stomal orifice 54 in the flange 50, although that orifice 54 need not necessarily be circular. There is a smooth upraised rim 58 around part of the exterior of the flange 50, on the side of the flange 50 which is to be secured to the ostomy bag in use, i.e., the surface 50a shown in FIG. 6 will be secured to the ostomy bag 5 in use, and the surface 50b is intended to contact adhesive on an adhesive annulus. The extension 52 has a central circular hole 56 which is aligned with a pre-punched hole in the adjacent wall of the ostomy bag 5 when in use.

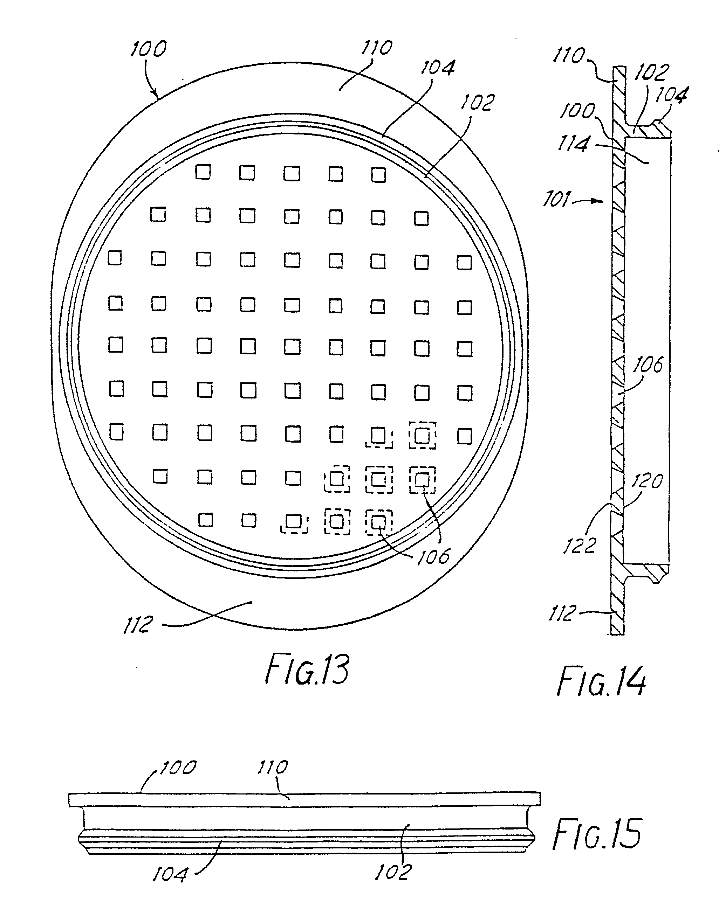

The extension 52 has a peripheral wall 60 containing an internal peripheral groove 62 therein. Spaced just inwardly of this wall 60 is a circular upraised rib 66 projecting from the base wall 68 of the extension 52. The configuration is best illustrated in FIG. 7. There is a bevel 70 on the wall 60 to facilitate insertion of a filter cover as will be better understood from a consideration of FIG. 7 in conjunction with FIG. 13 which will be described hereinafter.

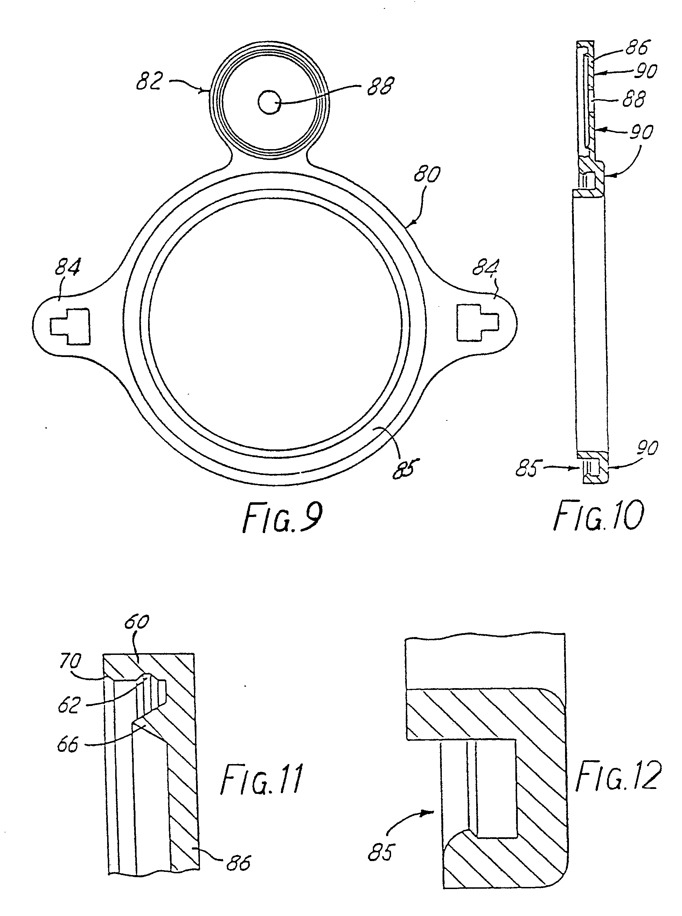

FIG. 8 is an enlargement of part of FIG. 6 which illustrates the provision of a shielding and guiding rim 74 whose purpose is to limit the possibility of any discharged faecal material becoming lodged in any crack or crevice which may exist where the bag joins the surface 50a.

The purpose of the groove 62 is to engage and receive a counterpart rim or rib on a filter cover as will be described hereinafter. The purpose of the rib 66 is to engage the adjacent surface of a flat circular disc filter which is to be accommodated in the extension 52 in such a way as to apply some compression effect at a circular line spaced inwardly from the exterior of the filter disc. This is to inhibit exiting gases from bypassing the filter.

Referring now to FIGS. 9-12, FIGS. 9 and 10 illustrate a bag-side coupling element 80 having an extension 82 and tabs 84 for the attachment of a belt, not shown. The bag-side coupling element 80 has a peripheral channel section 85 for receiving a counterpart body-side coupling element.

The extension 82 has a base wall 86 which has a central hole 88 therein. In use, the hole 88 is aligned with a pre-punched hole in the adjacent wall of an ostomy bag which is suitably secured to the coupling element 80 over the surfaces indicated at 90.

The extension 82, as shown in FIG. 11, has a detailed configuration similar to that shown in FIG. 7, that is to say there is an internal groove 62, an upraised rib 66, and a bevel 70. The purpose of these parts is the same as that described in connection with FIGS. 6 and 7.

The filter cover 101 illustrated in FIGS. 13 and 14, has a top wall 100, connected to a substantially cylindrical wall 102. The filter cover 101 is preferably made of plastic such as a high density polyethylene, but any material suitable for the "snap fit" connection may be used. The wall 102 has an external rib or detent 104 for engagement in the groove 62 described above. The top wall 100 has square holes 106 therein and is of substantially oval shape as seen in plan, having flanges 110, 112 which extend beyond the confines of the wall 102. The flanges 110, 112 are provided to enable a user to separate the filter cover 101 from the extension, e.g., by inserting a finger nail underneath the flange 110 and lifting. A shallow cylindrical space 114 accommodates a circular disc filter. Any known form of circular disc filter may be employed.

It has been found advantageous to provide exit holes 106 for the gas which are of tapered configuration, the walls of the holes tapering inwardly so that the hole cross-sectional area at the inner surface 120 (FIG. 13) of the wall 100 is less than the hole cross-sectional area at the outer surface 122 of the wall.

In use, the filter cover 101 can readily be attached to the extension 52 or 82 as the case may be, by pushing it on so that the snap fitting parts 104 and 62 interengage. A filter disc is normally placed within the space 114 before snapping the two parts together. When they are snapped together, the raised rib 66 pinches the filter at a circular location space just inwardly of its outer periphery thereby ensuring that any gas leaving the ostomy bag through the hole 56 or 88 and entering the filter housing defined by the interengaging walls 60 and 102 (or 82 and 102) is constrained to pass through the filter to reach the exit holes 106 and cannot by-pass the filter. A new filter can easily be inserted without removing the ostomy bag. Hence, there is no need to uncouple the body side and bag side couplings when changing a filter, nor any need to remove the body side attachment member from the peristomal skin region. Hence, undesired disturbance of this skin region is minimized.

As will be understood from the foregoing description, it is not essential that the coupling between the bag and the body-side pad be by way of flat flange and adhesive. As an alternative, any known ostomy coupling arrangement may be employed and in particular the coupling arrangement described in the patents cited above may be used. An advantage of the invention as particularly disclosed herein is that manufacture of ostomy bags is greatly simplified, it being possible to use the same bag-side coupling element for bags having filters as for bag which do not have filters.

## Claims

1. A bag side coupling element for an ostomy bag, comprising a flat, elongated member including a flange (50) surrounding a stomal orifice (54) and having a coupling portion by means of which it can be coupled to a body side coupling element, said flange having an extension (52) connected thereto, the extension (52) has a central hole (56) through which gas from the bag can enter, characterised in that the extension (52) has a peripheral wall (60) containing an internal peripheral groove (62) therein, there being spaced inwardly of the wall (60) a circular upraised rib (66) which projects from a base wall (68) of the extension (52), and in that the groove (62) is arranged to receive a counterpart rib or rim (104) on a substantially cylindrical wall (102) of a filter cover (101), a disc filter being locatable within the space defined by the extension (52) and its cover (101), whereby in use the disc filter is caused to bear on the upraised rib (66) so as to prevent the disc filter being by-passed by gases to be deodorised or filtered.

2. A coupling element according to claim 1 in which the filter cover (101) is a snap-in filter cover which snaps into a wall of the extension (52).

3. A coupling element according to claim 1 or 2 in which the flange (50) and its extension (52) are integrally molded from a single piece of plastics material.

4. A coupling element according to claims 1, 2 or 3 in which a disc filter is disposed within a housing whose peripheral wall is formed integrally with the extension.

5. A coupling element according to claim 1, 2, 3 or 4 in which the filter cover (101) has a plurality of holes (106) therein, the holes being of tapering configuration such that their cross-sectional area at the inner surface of the cover (101) is less than that at the outer surface of the cover (101).

6. A coupling element according to any preceding claim in which the filter cover (101) is of substantially oval shape and has flanges (110,112) which extend beyond the confines of the wall (102).

7. An ostomy bag including a bag-side coupling element according to any one of claims 1-6.

## Revendications

1. Elément de raccord côté sac pour sac de stomie, comprenant un élément plat et allongé qui comporte une bride (50) entourant un orifice stomal (54) et ayant une partie raccord au moyen de laquelle il peut être accouplé à un élément de raccord côté corps, un prolongement (52) étant raccordé à ladite bride, ce prolongement (52) étant percé d'un trou central (56) par lequel peut entrer le gaz venant du sac, caractérisé en ce que le prolongement (52) a une paroi périphérique (60) contenant une rainure périphérique interne (62), une nervure circulaire (66) qui fait saillie vers le haut à partir d'une paroi de base (68) du prolongement (52) se trouvant à une certaine distance à l'intérieur de la paroi (60), et en ce que la rainure (62) est disposée de façon à recevoir une nervure ou un rebord complémentaire (104) d'une paroi sensiblement cylindrique (102) d'un couvercle (101) de filtre, un disque filtrant pouvant être logé à l'intérieur de l'espace délimité par le prolongement (52) et par son couvercle (101), de sorte que, lorsque le dispositif est utilisé, le disque filtrant est amené à porter sur la nervure saillante (66) de manière à empêcher les gaz à désodoriser ou à filtrer de contourner le disque filtrant.

2. Elément de raccord selon la revendication 1, dans lequel le couvercle (101) de filtre est un couvercle de filtre à encliquetage qui s'encliquète dans une paroi du prolongement (52).

3. Elément de raccord selon la revendication 1 ou 2, dans lequel la bride (50) et son prolongement (52) sont moulés intégralement dans une seule pièce de matière plastique.

4. Elément de raccord selon la revendication 1, 2 ou 3, dans lequel un disque filtrant est disposé a l'intérieur d'un logement dont la paroi périphérique fait corps avec le prolongement.

5. Elément de raccord selon la revendication 1, 2, 3 ou 4, dans lequel le couvercle (101) de filtre est percé de plusieurs trous (106) ces trous ayant une forme tronconique telle que leur section droite au niveau de la surface intérieure du couvercle (101) est plus petite qu'au niveau de la surface extérieure du couvercle (101).

6. Elément de raccord selon l'une quelconque des revendications précédentes, dans lequel le couvercle (101) de filtre a une forme sensiblement ovale et comporte des rebords (110, 112) qui dépassent les limites de la paroi (102).

7. Sac de stomie comportant un élément de raccord côté sac selon l'une quelconque des revendications 1 à 6.

**Patentansprüche**

1. Beutelseitiges Anschlußelement für einen Ostomiebeutel mit einem flachen langgestreckten Glied, das einen Flansch (50), der eine Stomaöffnung (54) umgibt, und einen Anschlußabschnitt aufweist, durch den ein körperseitiges Anschlußelement angeschlossen werden kann, wobei der Flansch einen mit ihm verbundenen Fortsatz (52) mit einem zentralen Loch (56) aufweist, durch das Gas aus dem Beutel hindurchgehen kann, **dadurch gekennzeichnet**, daß der Fortsatz (52) eine periphere Wand (60) mit einer inneren peripheren Rille (62) aufweist, wobei von der Wand (60) nach innen beabstandet eine kreisförmige hervorstehende Rippe (66) vorgesehen ist, die aus einer Basiswand (68) des Fortsatzes (52) hervorragt, und daß die Rille (62) zur Aufnahme eines Gegenstücks in Form einer Rippe oder eines Reifens (104) auf einer im wesentlichen zylindrischen Wand (102) eines Filterdeckels (101) vorgesehen ist, wobei ein Scheibenfilter in dem Raum zwischen dem Fortsatz (52) und dem Filterdeckel (101) angeordnet ist und im Gebrauch der Scheibenfilter an der vorstehenden Rippe (66) anliegt, um zu verhindern, daß der Scheibenfilter von den Gasen umgangen wird, die deodorisiert oder gefiltert werden sollen.

2. Anschlußelement nach Anspruch 1, wobei der Filterdeckel (101) ein Schnappfilterdeckel ist, der in eine Wand des Fortsatzes (52) einschnappt.

3. Anschlußelement nach Anspruch 1 oder 2, wobei der Flansch (50) und sein Fortsatz (52) aus einem einzelnen Stück Kunststoffmaterial einstückig ausgebildet sind.

4. Anschlußelement nach Anspruch 1, 2 oder 3, wobei ein Scheibenfilter in einem Gehäuse angeordnet ist, dessen periphere Wand einstückig mit dem Fortsatz ausgebildet ist.

5. Anschlußelement nach einem der Ansprüche 1 bis 4, wobei der Filterdeckel (101) mehrere Löcher (106) aufweist, die konisch ausgebildet sind, so daß ihre Querschnittsfläche an der inneren Oberfläche des Deckels (101) kleiner als an der äußeren Oberfläche des Deckels (101) ist.

6. Anschlußelement nach einem der vorstehenden Ansprüche, wobei der Filterdeckel (101) eine im wesentlichen ovale Form und Flansche (110, 112) aufweist, die sich über die Begrenzung der Wand (102) hinaus erstrecken.

7. Ostomiebeutel mit einem beutelseitigen Anschlußelement nach einem der Ansprüche 1 bis 6.

FIG.1

FIG.2

FIG. 3

FIG. 4

FIG.5

FIG.6

FIG.7

FIG.8

FIG.9

FIG.10

FIG.11

FIG.12

FIG.13

FIG.14

FIG.15